(19) Europäisches Patentamt
European Patent Office
Office européen des brevets

(11) EP 0 806 435 B1

(12) **EUROPÄISCHE PATENTSCHRIFT**

(45) Veröffentlichungstag und Bekanntmachung des Hinweises auf die Patenterteilung:
**09.01.2002 Patentblatt 2002/02**

(51) Int Cl.[7]: **C08B 37/08**, A61K 7/00, A61K 7/06, A61K 7/11

(21) Anmeldenummer: **97107280.6**

(22) Anmeldetag: **02.05.1997**

(54) **O-Hydroxyalkylchitine und deren Verwendung in kosmetischen Mitteln**

O-hydroxyalkyl chitins and their use in cosmetics

Chitine O-hydroxyalkylée et son utilisation dans les agents cosmétiques

(84) Benannte Vertragsstaaten:
**DE ES FR GB IT**

(30) Priorität: **11.05.1996 DE 19619111**

(43) Veröffentlichungstag der Anmeldung:
**12.11.1997 Patentblatt 1997/46**

(73) Patentinhaber: **Wella Aktiengesellschaft**
**64274 Darmstadt (DE)**

(72) Erfinder:
- **Titze, Hans-Jürgen**
  **64401 Gross-Bieberau (DE)**
- **Steinbrecht, Karin, Dr.**
  **64372 Ober-Ramstadt (DE)**
- **Birkel, Susanne, Dr.**
  **64380 Rossdorf (DE)**
- **Franzke, Michael, Dr.**
  **64380 Rossdorf-Gundernhausen (DE)**
- **Borth, Silvia**
  **64407 Fränkisch-Crumbach (DE)**

(56) Entgegenhaltungen:
**EP-A- 0 507 272**

- **CHEMICAL ABSTRACTS, vol. 124, no. 15, 9.April 1996 Columbus, Ohio, US; abstract no. 202813, "Synthesis and properties of water soluble O-(2-hydroxypropyl) chitin" XP002052211 & WANG AIQIN ET AL: TIANRAN CHANWU YANJIU YU KAIFA, Bd. 7, Nr. 3, 1995, CHINA, Seiten 79-84,**
- **SEON JEONG KIM ET AL.: "Synthesis and characterization of ether-type chitin derivatives" MACROMOLECULAR CHEMISTRY AND PHYSICS, Bd. 195, Nr. 5, 1994, USA, Seiten 1687-1693, XP002052210**
- **DATABASE WPI Week 8431 Derwent Publications Ltd., London, GB; AN 84-191015 XP002052212 & JP 59 106 409 A (ICHIMARU PHARCOS INC) , 20.Juni 1984**
- **PATENT ABSTRACTS OF JAPAN vol. 096, no. 003, 29.März 1996 & JP 07 304626 A (KAWAKEN FINE CHEM CO LTD;OTHERS: 01), 21.November 1995,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 806 435 B1

## Beschreibung

Die Erfindung betrifft die Verwendung von mindestens einem O-Hydroxyalkylchitin in Mitteln zur Festigung der Haare, ein Verfahren zur Herstellung von O-Hydroxyalkylchitin sowie das nach diesem Verfahren hergestellte O-Hydroxyalkylchitin.

Mittel zur Festigung der Haare bestehen üblicherweise aus Lösungen von filmbildenden synthetischen oder auch natürlichen Polymeren. Als synthetische Polymere kommen beispielsweise Polyvinylpyrrolidon, Polyvinylacetat, Polyacrylsäure oder Polymethacrylsäurepolymerisate in Betracht. Von natürlichen Polymeren finden z.B. Schellack, Gelatine, Polysaccharide und deren Derivate, Cellulose und Chitosansalze Verwendung.

Die in Haarfestigungsmitteln mit einer stark festigenden Wirkung eingesetzten synthetischen und natürlichen Polymere führen oftmals zu unerwünschten Nebenwirkungen wie beispielsweise einem rauhen Griff der Haare.oder einer übermäßigen Rückstandsbildung auf dem Haar. Somit fehlt bei einer guten Festigung der Haare der natürliche Griff, das heißt, Haar fühlt sich nicht wie natürliches Haar an.

Des weiteren sind die oben genannten Polymere in der Regel schlecht wasserlöslich.

Es ist bereits bekannt, Chitosane zu derivatisieren, um sie wasserlöslich zu machen und in Mitteln zur Festigung der Haare einzusetzen. Hierzu zählen beispielsweise Hydroxypropylchitosane (vgl. DE 35 01 891, DE 35 41 305) und Hydroxybutylchitosane (vgl. DE 36 14 697). Chitosane werden in der Regel durch Salzbildung in Wasser gelöst. Dies hat den Nachteil, daß hierdurch die Verträglichkeit mit anionischen Polymeren verschlechtert wird und auch die festigende Wirkung abnimmt.

Weiterhin sind die bekannten Hydroxyalkylchitosane aufwendig in der Herstellung, da zunächst Chitin zu Chitosan entacetyliert und in einem weiteren Verfahrensschritt zu dem gewünschten Chitosanderivat umgesetzt wird.

Aus der JP-OS 59-106 409 ist es bekannt, Hydroxyethylchitin als Pflegewirkstoff in Haut- und Haarbehandlungsmitteln einzusetzen. Ein Hinweis auf Haarfestigungsmittel mit einem Gehalt an Hydroxyethylchitin oder höheren Hydroxyalkylchitinen ist diesem Dokument nicht zu entnehmen. Weiterhin erwiesen sich die Eigenschaften des nach dem Verfahren der JP-OS 59 106 409 erhältlichen Hydroxyethylchitins als unzureichend für eine Verwendung in Mitteln zur Festigung von Haaren.

Die EP 0 507 272 A1 beschreibt kosmetische Haarbehandlungsmittel mit einem Gehalt an bestimmten, wasserlöslichen Chitinderivaten. Als geeignete Chitinderivate werden u.a. Hydroxyalkylenchitine genannt.

Der Abstract CA 124:202813 beschreibt ein Herstellverfahren für O-(2-Hydroxypropyl)chitin. Ein weiteres Herstellverfahren für Hydroxypropylchitin ist in Macromol. Chem. Phys. 195, 1687-1693 (1994) beschrieben.

In der JP 07-304,626 wird ein synergistischer Effekt in bezug auf ein gesteigertes Wasserrückhaltevermögen eines kosmetischen Mittels, welches eine Kombination eines bestimmten N-Carboxypropanoyl-O-carboxymethylchitosans und bestimmten wasserlöslichen Chitinderivaten enthält, beschrieben.

Es bestand somit die Aufgabe, ein geeignetes, filmbildendes und haarfestigendes Polymer zur Verfügung zu stellen, welches leicht synthetisch zugänglich und ausreichend wasserlöslich ist und bei Verwendung in einem Haarfestigungsmittel einen natürlichen Griff und eine gute Festigung des behandelten Haares sowie ein gutes Rückstandsverhalten bewirkt.

Es wurde nun gefunden, daß die Verwendung von mindestens einem O-Hydroxyalkylchitin mit monomeren Einheiten der allgemeinen Formel

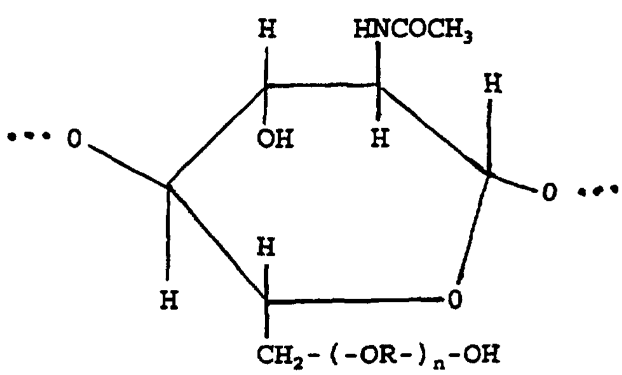

wobei R für eine gerade oder verzweigte Alkylengruppe mit 3 bis 20 Kohlenstoffatomen und n für eine Zahl größer oder gleich 1 steht, in Mitteln zur Festigung von Haaren die gestellten Anforderungen in hervorragender Weise erfüllt.

Die erfindungsgemäßen O-Hydroxyalkylchitine sind ohne Salzbildung gut wasserlöslich, haben sehr gute

filmbildende und haarfestigende Eigenschaften, zeigen ein sehr gutes Rückstandsverhalten auf damit behandelten Haaren, sind mit kationischen und anionischen Polymeren gut verträglich, sind physiolgisch unbedenklich und biologisch abbaubar und das mit ihnen behandelte Haar weist einen angenehmen, natürlichen Griff und eine gute Festigung auf. Weiterhin sind die O-Hydroxyalkylchitine sehr gut als Verdickungsmittel und zur Herstellung von Gelen zu verwenden. So führt beispielsweise der Zusatz von niederen Alkoholen wie Ethanol zu Lösungen von O-Hydroxyalkylchitin zu einer starken Viskositätserhöhung bzw. einer Verdickung bis hin zur Gelbildung.

Daher sind die erfindungsgemäßen Hydroxyalkylchitine besonders gut geeignet um als Verdicker in kosmetischen Mitteln verwendet zu werden. Ein Einsatz empfiehlt sich beispielsweise in Dauerwellmitteln, Haarfärbemitteln, Shampoos, Haarkuren, Duschbädern, Lippenstiften, Seifen, Enthaarungscremes, Hautcremes, Nagelbettcremes, Peelingcremes, Augenpflegeprodukten und Rasierschäumen.

In einer bevorzugten Ausführungsform enthält der Erfindungsgegenstand mindestens ein O-Hydroxyalkylchitin mit einer Hydroxyalkylgruppe mit 3 bis 6 Kohlenstoffatomen, besonders bevorzugt ist O-Hydroxypropylchitin und O-Hydroxybutylchitin. Es können auch Gemische von O-Hydroxyalkylchitinen mit Hydroxyalkylresten unterschiedlicher Kettenlänge eingesetzt werden. Auch kann ein und dasselbe Chitinmolekül mit Hydroxyalkylresten unterschiedlicher Kettenlänge substituiert sein. Bevorzugt sind weiterhin O-Hydroxyalkylchitine mit einem Acetylierungsgrad am Stickstoff von größer 0,95 und einem Wert für den Substitutionsgrad n von größer 1, vorzugsweise zwischen 1,5 und 10, besonders bevorzugt zwischen 2 und 6.

Ein erfindungsgemäßes Mittel zur Festigung der Haare weist vorzugsweise einen Gehalt an 0,01 bis 20 Gewichtsprozent, besonders bevorzugt 0,02 bis 2 Gewichtsprozent mindestens eines O-Hydroxyalkylchitins in einer geeigneten kosmetischen Grundlage auf.

Da die erfindungsgemäßen O-Hydroxyalkylchitine hervorragend verträglich sind mit weiteren Polymeren, insbesondere auch ionischen, das heißt kationischen, anionischen und amphoteren Polymeren, ist eine weitere, bevorzugte Ausführungsart des Erfindungsgegenstandes ein kosmetisches Mittel mit einem Gehalt an

a) 0,01 bis 20 Gewichtsprozent mindestens eines erfindungsgemäßen O-Hydroxyalkylchitins und

b) 0,001 bis 5 Gewichtsprozent, besonders bevorzugt 0,05 bis 2 Gewichtsprozent mindestens eines weiteren natürlichen oder synthetischen, nicht ionischen, anionischen, amphoteren oder kationischen Polymers.

Das erfindungsgemäße Mittel kann zusätzlich die folgenden, in kosmetischen Mitteln üblichen Zusatzstoffe enthalten:

Andere Polymere wie z.B. anionische, kationische, amphotere oder nichtionische Polymere synthetischen oder natürlichen Ursprungs in einer Menge von 0,01 bis 20 Gewichtsprozent; Neutralisierungsmittel wie z.B. 2-Amino-2-Methyl-1-Propanol, 2-Amino-1-Butanol und Tri-isopropylamin; Lösungsmittel beispielsweise niedere Alkane mit einer Kettenlänge bis zu 12 $CH_2$-Gruppen, höhere Alkane (Kettenlänge größer als 12 $CH_2$-Gruppen), Paraffine, Glykolether und geradkettige und verzweigte Alkohole mit einer Kettenlänge bis zu 5 Kohlenstoffatomen; anionische, kationische, amphotere oder nichtionische Tenside in einer Menge von 0,1 bis 30 Gewichtsprozent; Verdicker wie Polyethylenglykole oder Kokosfettsäurediethanolamid in einer Menge von 0,2 bis 3,0 Gewichtsprozent; Emulgatoren, Dispersionsmittel, Stabilisatoren, Konservierungsmittel, Treibgase wie Fluorkohlenwasserstoffe, niedere Alkane, komprimierte Gase und Dimethylether, physikalische oder chemische UV-Absorber, Weichmacher, Bindemittel, Fließverbesserer, Desinfektionsmittel, Antistatika, Öle, Antioxidantien, Wachse, Fruchtwachse, Bleichmittel, Milderungsmittel, Antiirritantien, Konservierungsmittel, Trägermaterialien, Fettalkohole, Spreithilfsmittel, Enzyme, planzliche Extrakte, Entschäumer, Parfümöle, Kämmbarkeitsverbesserer, Oxidations- und Reduktionsmittel, Vitamine, Anfärbefarbstoffe, Haarfärbefarbstoffe (direktziehende und Reaktivfarbstoffe), Pro-Vitamine, Proteine, Hydrolysate, Feuchthaltemittel, Konditionierer, Wirkstoffe wie beispielsweise Radikalfänger oder Anti-Schuppen-Wirkstoffe, Rückfetter und Glanzgeber.

Das erfindungsgemäße Mittel kann unter Verwendung eines Treibmittels oder mit Hilfe einer mechanisch betriebenen Sprühvorrichtung versprüht werden oder mit Hilfe einer Schaumerzeugungsvorrichtung als Schaum abgegeben werden.

Die erfindungsgemäß einzusetzenden hydroxyalkylierten Chitine werden aus hochmolekularem Chitin, beispielsweise Krabbenchitin, gewonnen. Das mittlere Molekulargewicht beträgt vorzugsweise 100.000 bis 2.000.000 g/mol, besonders bevorzugt mindestens 400.000 g/mol.

Hydroxyalkylchitine können erhalten werden, indem man Chitin in einem Alkalisierungsmittel, vorzugsweise 1 bis 43 prozentiger Natriumhydroxidlosung rührt, durch mindestens einmaliges, vorzugsweise mehrmaliges Einfrieren auf bis zu -78°C und Auftauen in Alkalichitin überführt, welches anschließend abgetrennt und mit Alkylenoxiden, beispielsweise Propylenoxid oder Butylenoxid für 0,5 bis 72 Stunden, vorzugsweise für 3 bis 50 Stunden bei Temperaturen zwischen 30 und 100°C, bevorzugt zwischen 30 und 50°C umgesetzt wird. Das molare Verhältnis zwischen Chitin und Alkyloxiden liegt vorzugsweise zwischen 1:2 und 1:40.

Nach beendeter Reaktion entfernt man das überschüssige Alkylierungsmittel, trennt eventuell vorhandene

unlösliche Anteile aus den Lösungen des Chitinderivates durch Filtration ab, neutralisiert gegebenenfalls, engt am Rotationsverdampfer ein, löst in Wasser und entsalzt mittels Membranfiltration. Das reine Endprodukt erhält man aus der wäßrigen Lösung durch Gefriertrocknen.

Ein weiterer Gegenstand der Erfindung ist ein Verfahren zur Herstellung von Hydroxyalkylchitinen in welchem (a) Chitin gleichzeitig mit einem Überschuß an einem Alkalisierungsmittel und einem Überschuß an einem Alkylenoxid umgesetzt wird, wobei der Alkylrest des Alkylenoxids aus 2 bis 20 Kohlenstoffatomen besteht und (b) das Reaktionsprodukt in üblicher Weise isoliert und gereinigt wird.

Die Reaktionszeit beträgt vorzugsweise 0,5 bis 72 Stunden bei 30 bis 100°C. Das molare Verhältnis zwischen Chitin und Alkylenoxid beträgt vorzugsweise 1:2 bis 1:40. Es kann auch ein Gemisch von verschiedenen Alkylenoxiden eingesetzt werden. Vorzugsweise werden Alkylenoxide mit 3 bis 6 Kohlenstoffatomen eingesetzt. Besonders bevorzugt sind Propylenoxid und Butylenoxid.

Die nachfolgenden Beispiele sollen die Erfindung näher erläutern.

**Beispiel 1:** Zweistufige Synthese von Hydroxyalkylchitin

<u>Herstellung von Alkalichitin</u>

21,2 g Chitin (0,1 Mol) mit einem Molekulargewicht von 400.000 g/mol werden in 325 ml Natronlauge (43%ig) aufgeschlämmt und 30 Minuten bei -78°C eingefroren. Nach 60 Minuten Stehenlassen bei Raumtemperatur wird dieser Vorgang noch zwei mal wiederholt. Anschließend läßt man über Nacht auftauen und saugt auf ein Gesamtgewicht von ca. 160 g ab.

<u>Herstellung von Hydroxypropylchitin</u>

Die vorhergehende Menge (160 g) Alkalichitin/NaOH läßt man mit 250 ml (3,6 mol) Propylenoxid unter Rückfluß 24 h lang reagieren. Nach Abdestillieren des über-schüssigen Propylenoxids, löst man in 2 l Wasser, filtriert gegebenenfalls ab und neutralisiert mit Salzsäure. Nach der Dialyse mittels Membranfiltration wird das reine Hydroxypropylchitin durch Gefriertrocknung gewonnen.

Die Ausbeute beträgt ca. 25 g. Aussehen: weiße Substanz, welche hochviskose, klare Lösungen ergibt. Das Molekulargewicht beträgt $1{,}15 \cdot 10^6$ g/mol. Der Acety- lierungsgrad am Stickstoff ist ≥ 98,5 %. Der Hydroxypropylierungsgrad beträgt 2,66 pro Monomereinheit.

Bei Zugabe von Ethanol zu einer 1%igen wäßrigen Lösung entsteht ein Gel.

<u>Herstellung von Hydroxybutylchitin</u>

Durch Umsetzung von 320 g Alkalichitin/NaOH mit 610 ml Butylenoxid bei 40 °C werden nach dem gleichen Verfahren 37 g Hydroxybutylchitin als weiße, klar wasserlösliche Substanz erhalten.

<u>Herstellung von Hydroxyethylchitin</u>

Durch Umsetzung von 115 g Alkalichitin/NaOH mit 440 ml (225 g) Ethylenoxid in einem Druckbehälter und langsamen Erwärmen von 0 °C auf 30 °C werden 10,4 g Hydroxyethylchitin erhalten. Da die Reaktion nach der Startphase exotherm verläuft, ist eine starke Gegenkühlung erforderlich. Nach einem Druckanstieg auf 6 bar und einem Temperaturanstieg auf 85 °C fällt der Druck nach 25 Stunden wieder auf 0 bar und die Temperatur auf 30 °C.

Das nach Gefriertrocknung erhaltene, weiße Hydroxyethylchitin ergibt in Wasser hochviskose, klare Lösungen.

**Beispiel 2:** Einstufige Synthese von Hydroxy-propylchitin

42,6 g Chitin (0,2 Mol) mit einem Molekulargewicht von 400.000 oder 1.200.000 g/mol läßt man zusammen mit 120 ml Natronlauge und 500 ml (7,2 Mol) Propylenoxid unter gutem Rühren und unter Rückfluß 24 h reagieren. Nach Abdestillieren des überschüssigen Propylenoxids löst man in 4 l Wasser, filtriert gegebenenfalls ab und neutralisiert mit Salzsäure. Nach der Dialyse mittels Membranfiltration wird das reine Hydroxypropylchitin durch Gefriertrocknung gewonnen.

Die Ausbeute beträgt ca. 54 g.

**Beispiel 3:** Festigerlotion

| | |
|---|---|
| 0,25 g | Hydroxypropylchitin nach Beispiel 1 |
| 0,50 g | Propylenglykol |
| 0,15 g | Parfümöl |
| 0,10 g | Methylparaben |
| 0,10 g | Propylparaben |
| 98,90 g | Wasser |
| 100,00 g | |

**Beispiel 4:** Schaumfestiger

| | |
|---|---|
| 0,2 g | Hydroxypropylchitin nach Beispiel 1 |
| 0,3 g | Cetyltrimethylammoniumchlorid |
| 0,2 g | Parfümöl |
| 0,2 g | 1,2-Dibrom-2,4-dicyanobutan |
| 99,1 g | Wasser |
| 100,00 g | |

Die Mischung wird in einen Aluminiumaerosolbehälter mit einem Verhältnis von Wirkstoffmischung zu Butan von 92:8 abgefüllt.

Beispiel 5: Gel mit haarfestigender Wirkung

| | |
|---|---|
| 0,15 g | Hydroxypropylchitin nach Beispiel 1 |
| 0,30 g | Hydroxypropylcellulose mit einem mittleren Molekulargewicht von 1.150.000 g/mol |
| 0,15 g | Parfümöl |
| 20,00 g | Ethanol |
| 79,40 g | Wasser |
| 100,00 g | |

**Beispiel 6:** Colorfestiger

| | |
|---|---|
| 0,1000 g | Hydroxypropylchitin nach Beispiel 1 |
| 2,1000 g | Vinylpyrrolidon/Vinylacetat Copolymer |
| 0,2000 g | Parfüm |
| 0,0700 g | 1-Amino-4-(2',3'-dihydroxypropyl)amino-5-chlor-2-nitrobenzol |
| 0,0500 g | Basic Brown 17 (C.I. 12 251) |
| 0,0100 g | Basic Blue 7 (C.I. 42 595) |
| 0,0023 g | Basic Violett 14 (C.I. 42 510) |
| 47,4677 g | Wasser |
| 50,0000 g | Ethanol |
| 100,000 g | |

**Beispiel 7:** Flüssigfestiger

| | |
|---|---|
| 0,6 g | Hydroxypropylchitin nach Beispiel 1 |
| 0,5 g | Vinylpyrrolidon/Dimethylaminoethylmethacrylat Copolymer |
| 0,1 g | Parfüm |
| 16,0 g | Ethanol |
| 82,8 g | Wasser |
| 100,0 g | |

**Beispiel 8:** Festigerlotion

| | |
|---|---|
| 0,30 g | Hydroxypropylchitin nach Beispiel 1 |
| 1,50 g | Vinylacetat/Crotonsäure/Vinylneodecanoat Copolymer |
| 0,50 g | Betain Monohydrat |
| 0,20 g | 1,2-Dibrom-2,4-dicyanobutan |
| 0,15 g | Parfüm |
| 97,35 g | Wasser |
| 100,00 g | |

**Beispiel 9**: Schaumfestiger

| | |
|---|---|
| 0,2 g | Hydroxypropylchitin nach Beispiel 1 |
| 1,0 g | t-Octylacrylamid/Acrylsäure/t-Butyl aminoethylmethacrylat Copolymer |
| 0,2 g | Decylpolyglucose |
| 0,2 g | Polypropylenglykol-(1)-Polyethylenglykol(9)-laurylglykolether |
| 0,2 g | 2-Amino-2-methyl-1-propanol |
| 0,1 g | Parfüm |
| 98,1 g | Wasser |
| 100,00 g | |

Die Wirkstofflösung wird im Verhältnis 9:1 mit einem Treibgasgemisch aus Propan/Butan abgefüllt.

**Beispiel 10:** Schaumfestiger

| | |
|---|---|
| 0,50 g | Hydroxypropylchitin nach Beispiel 1 |
| 0,75 g | Polyvinylpyrrolidon |
| 0,75 g | Vinylpyrrolidon/Vinylacetat Copolymer |
| 0,30 g | Cetyltrimethylammoniumchlorid |
| 0,20 g | Parfüm |
| 97,50 g | Wasser |
| 100,00 g | |

Die Wirkstofflösung (90 %) wird zusammen mit den Treibgasen Propan/Butan (18 %) und Dimethylether (2 %) abgefüllt.

**Beispiel 11:** Pumpspray (55 % VOC)

| | |
|---|---|
| 0,05 g | Hydroxypropylchitin nach Beispiel 1 |
| 1,68 g | Polyacrylsäure |
| 0,18 g | 2-Amino-2-methyl-1-propanol |
| 0,10 g | Parfüm |
| 42,99 g | Wasser |
| 55,00 g | Ethanol |
| 100,00 g | |

**Beispiel 12:** Aerosol-Haarspray

| | |
|---|---|
| 0,10 g | Hydroxypropylchitin nach Beispiel 1 |
| 7,50 g | Vinylacetat/Vinylpropionat/Crotonsäure Copolymer |
| 0,15 g | Parfüm |
| 45,00 g | Ethanol oder Isopropanol |
| 47,25 g | Wasser |
| 100,00 g | |

**Beispiel 13:** Vergleichsversuche zur haarfestigenden Wirkung: Wellstabilität

Es wurden fünf haarfestigende Mittel untersucht mit den folgenden Zusammensetzungen:

| | A | B | C | D | E |
|---|---|---|---|---|---|
| Hydroxypropylchitin gemäß Beispiel 1 | 0,500 g | - | - | - | - |
| Vinylpyrrolidon/Vinylacetat Copolymer | - | 0,500 g | - | - | - |
| Vinylpyrrolidon/Dimethylaminoethylmethacrylat Copolymer | - | - | 0,500 g | - | - |
| | A | B | C | D | E |
| Hydroxybutylchitosan | - | - | - | 0,500 g | - |
| Hydroxypropylchitosan | - | - | - | - | 0,500 g |
| Ethanol | 14,925 g | 14,925 g | 14,925 g | 14,925 g | 14,925 g |
| Wasser | 84,575 g | 84,575 g | 84,575 g | 84,575 g | 84,575 g |

Standardisierte Haarsträhnen mit 100 Haaren pro Strähne und mit einheitlicher Länge und Gewicht wurden in eine Folientasche gelegt, mit Wasser beträufelt und anschließend mit 60 Mikroliter Festigerlösung behandelt. Nach 10 Minuten wurden die behandelten Strähnen auf Wickler aufgewickelt und bei 70°C getrocknet. Danach wurden die Wickler für 1 Stunde in einem Exsikkator über Calciumchlorid getrocknet. Die gewellten Haarsträhnen wurden von den Wicklern entfernt und bei 20° Celsius und 65 % relativer Luftfeuchtigkeit ausgehängt. Die Länge der gewellten Haarsträhnen wurde vor dem Aushängen sowie nach 5 Stunden und 23 Stunden Aushängen gemessen. Die Wellstabilität W ergibt sich aus der Länge der ungewellten Strähnen L, der Länge der gewellten Strähnen vor dem Aushängen L (t=0) und der Länge der gewellten Strähnen nach dem Aushängen L (t): W = [L - L (t)] / [L - L (t=0)] • 100 %.
Es wurden die folgenden Wellstabilitäten gemessen:

| | A | B | C | D | E |
|---|---|---|---|---|---|
| Wellstabilität nach 5 Stunden | 59,5 % | 52,2 % | 59,0 % | 46,4 % | 46,0 % |
| Wellstabilität nach 23 Stunden | 51,2 % | 42,5 % | 48,3 % | 36,2 % | 36,3 % |

Die mit der erfindungsgemäßen Zusammensetzung A behandelten Haarsträhnen zeigen sowohl nach 5 Stunden als auch nach 23 Stunden eine höhere Wellstabilität als die mit den Vergleichszusammensetzungen B bis E behandelten Strähnen.

**Beispiel 14:** Vergleichsversuche zur haarfestigenden Wirkung: Bruchkraftmessung

Es wurden die gleichen Zusammensetzungen A bis E untersucht wie in Beispiel 13.
Standardisierte Haarsträhnen mit 100 Haaren pro Strähne und einheitlicher Länge und Gewicht wurden mit je 50 Mikroliter Festigerlösung beträufelt. Anschließend wurden die behandelten Strähnen bei 20° Celsius und 65 % relativer Luftfeuchtigkeit für 12 Stunden getrocknet. Danach erfolgten je drei Messungen, aus denen ein Mittelwert gebildet wurde, zur Bestimmung der Bruchkraft, die erforderlich ist, um den auf den Haarsträhnen vorhandenen Polymerfilm zu brechen. Es wurden die folgenden Bruchkräfte gemessen:

| | A | B | C | D | E |
|---|---|---|---|---|---|
| Bruchkraft [N] | 0,126 | 0,053 | 0,109 | 0,059 | 0,058 |

Die mit der erfindungsgemäßen Zusammensetzung A behandelten Haarsträhnen zeigen den höchsten Wert für die Bruchkraft gegenüber den mit den Vergleichszusammensetzungen B bis E behandelten Strähnen.

**Beispiel 15:** Substitutionsgrade der Hydroxyalkylchitine

Die erfindungsgemäß hergestellten Hydroxyalkylchitine wiesen die folgenden Hydroxyalkylierungsgrade n pro Monomereinheit auf:

| **Substanz** | **Molekulargewicht des Ausgangschitins** | **Herstellungsverfahren** | **Hydroxyalkylierungsgrad** |
|---|---|---|---|
| Hydroxyethylchitin | 400.000 g/mol | analog Beispiel 1 | 2,2 |
| Hydroxypropylchitin | -"- | -"- | 2,7 |
| -"- | 1.200.000 g/mol | -"- | 3,2 |
| - "- | 400.000 g/mol | analog Beispiel 2 | 5,1 |
| -"- | 1.200.000 g/mol | -"- | 3,0 |
| Hydroxybutylchitin | 1.200.000 g/mol | analog Beispiel 2 | 3,8 |

**Beispiel 16:** Viskositätsmessungen

Es wurden 0,5%ige Lösungen von Hydroxyalkylchitinen in Wasser hergestellt. Je 20 g dieser Lösungen wurden mit 20 g Ethanol verrührt. Die Fließgrenzen und Viskositäten wurden vor und nach dem Ethanolzusatz gemessen. Die Messungen erfolgten bei 25°C mit einem Bohlin CS Rheometer. Die Ergebnisse sind in der folgenden Tabelle zusammengefaßt:

| Substanz | Hydroxyehtylchitin | Hydroxypropylchitin | Hydroxypropylchitin |
|---|---|---|---|
| Molekulargewicht des Ausgangschitins | 1.200.000 g/mol | 1.200.000 g/mol | 1.200.000 g/mol |
| Herstellungsverfahren | analog Beispiel 1 | analog Beispiel 1 | analog Beispiel 2 |
| Fließgrenze in Pa einer 0,5%-igen Lösung in Wasser | 0,118 | 0,118 | 0,117 |
| Fließgrenze in Pa nach 1:1 Verdünnung mit Ethanol | 0,118 | 5,87 | 5,06 |
| Viskosität in mPa.s einer 0,5%igen Lösung in Wasser | 10,2 | 28,8 | 22,4 |
| Viskosität in mPa.s nach 1:1 Verdünnung mit Ethanol | 27,3 | 320 | 400 |

Die Versuche zeigen, daß auf einfache Weise eine deutliche Viskositätserhöhung durch Verdünnung einer wäßrigen Lösung der Hydroxyalkylchitine mit Ethanol erreicht werden kann.

**Beispiel 17:** Schaumfestiger

| | A | B | C |
|---|---|---|---|
| Hydroxybutylchitin gemäß Beispiel 1 | 0,50 g | - | - |
| Hydroxypropylchitin (gemäß Beispiel 2, M = 400.000 g/mol) | - | 0,50 g | - |
| Hydroxypropylchitin (gemäß Beispiel 2, M = 1.200.000 g/mol) | - | - | 0,50 g |
| Tetraoxyethylenlaurylether | 0,50 g | 0,50 g | 0,50 g |
| Parfüm | 0,20 g | 0,20 g | 0,20 g |
| Cetyltrimethylammoniumchlorid | 0,08 g | 0,08 g | 0,08 g |
| Ethanol | 10,00 g | 10,00 g | 10,00 g |
| Wasser | 88,72 g | 88,72 g | 88,72 g |
| | 100,00 g | 100,00 g | 100,00 g |
| pH | 6,5 | 6,4 | 5,7 |

**Beispiel 18:** Haarspray

| | |
|---|---|
| 1,10 g | Hydroxybutylchitin nach Beispiel 1 |
| 5,00 g | t-Octylacrylamid/Acrylsäure/t-Butylaminoethylmethacrylat Copolymer |
| 0,91 g | 2-Amino-2-methyl-1-propanol |
| 10,00 g | Wasser |
| 23,99 g | Ethanol |
| 60,00 g | Dimethylether |
| 100,00 g | |

**Beispiel 19:** Pflegefestiger

| | A | B | C |
|---|---|---|---|
| Hydroxybutylchitin (nach Beispiel 1) | 0,625 g | - | - |
| Hydroxypropylchitin (nach Beispiel 2, M = 400.000 g/mol) | - | 0,625 g | - |
| Hydroxypropylchitin (nach Beispiel 2, M = 1.200.000 g/mol) | - | - | 0,625 g |
| Vinylpyrrolidon/ Dimethylaminoethylmethacrylat Copolymer | 0,150 g | 0,150 g | 0,150 g |
| Cetyltrimethylammoniumchlorid | 0,100 g | 0,100 g | 0,100 g |
| Parfüm | 0,200 g | 0,200 g | 0,200 g |
| Ethanol | 15,000 g | 15,000 g | 15,000 g |
| Wasser | 83,925 g | 83,925 g | 83,925 g |
| | 100,000 g | 100,000 g | 100,000 g |

Sämtliche angegebenen Prozentzahlen stellen Gewichtsprozente dar, sofern nichts anderes vermerkt ist.

## Patentansprüche

1. Kosmetisches Mittel mit einem Gehalt an mindestens einem O-Hydroxyalkylchitin mit monomeren Einheiten der allgemeinen Formel

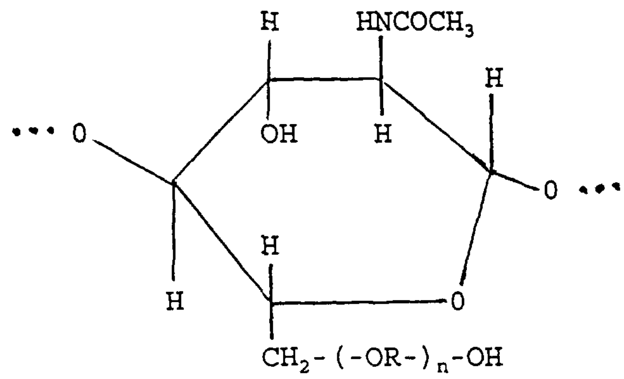

wobei R für eine gerade oder verzweigte Alkylengruppe mit 3 bis 20 Kohlenstoffatomen und n für eine Zahl größer oder gleich 1 steht, in einer geeigneten kosmetischen Grundlage, ausgenommen einem kosmetischen Mittel mit einem Gehalt an einer Kombination von (A) N-(3-Carboxypropanoyl-6-O-Carboxymethylchitosan und (B) einem wasserlöslichen Chitinderivat ausgewählt aus Hydroxypropylchitin oder Polyoxyethylen-polyoxypropylen-chitin, wobei das Verhältnis (A):(B) von 9,5:0,5 bis 0,5:9,5 und die Gesamtmenge von (A) und (B) von 0,001 bis 5 Gew.% beträgt.

2. Mittel nach Anspruch 1, **dadurch gekennzeichnet, dass** R für eine gerade oder verzweigte Alkylengruppe mit 3 bis 6 Kohlenstoffatomen steht.

3. Mittel nach einem der vorhergehenden Ansprüche, **dadurch gekennzeichnet, dass** n für eine Zahl von 1,5 bis 10 steht

4. Mittel nach einem der vorhergehenden Ansprüche, **dadurch gekennzeichnet, dass** das O-Hydroxyalkylchitin in einer Menge von 0,01 bis 20 Gew.% enthalten ist.

5. Mittel nach einem der vorhergehenden Ansprüche, **dadurch gekennzeichnet, dass** es zusätzlich 0,001 bis 5 Gew.% mindestens eines natürlichen oder synthetischen, nichtionischen, anionischen, amphoteren oder kationischen Polymeren enthält.

6. Verwendung von mindestens einem O-Hydroxyalkylchitin mit monomeren Einheiten der allgemeinen Formel gemäß Anspruch 1 in Mitteln zur Festigung von Haaren.

## Claims

1. Cosmetic composition with a content of at least one O-hydroxyalkylchitin with monomer units of the general formula

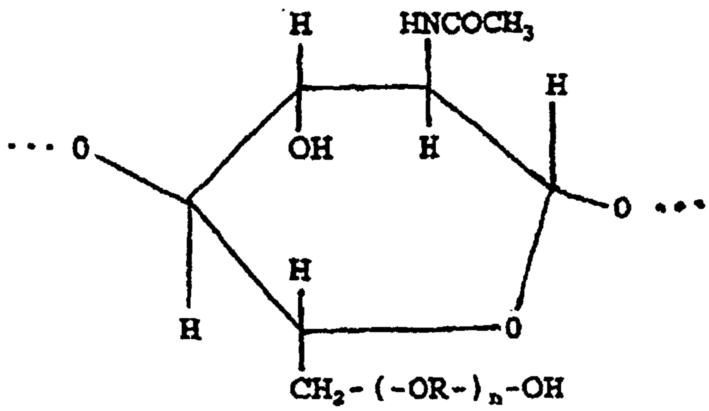

where R is a straight or branched alkylene group having 3 to 20 carbon atoms and n is a number greater than or equal to 1, in a suitable cosmetic base, with the exception of a cosmetic composition with a content of a combination of (A) N-(3-carboxypropanoyl-6-O-carboxymethylchitosan and (B) a watersoluble chitin derivative chosen from hydroxypropylchitin or polyoxyethylene-polyoxypropylenechitin, where the (A):(B) ratio is from 9.5:0.5 to 0.5:9.5 and the total amount of (A) and (B) is from 0.001 to 5% by weight.

2. Composition according to Claim 1, **characterized in that** R is a straight or branched alkylene group having 3 to 6 carbon atoms.

3. Composition according to one of the preceding claims, **characterized in that** n is a number from 1.5 to 10.

4. Composition according to one of the preceding claims, **characterized in that** O-hydroxyalkylchitin is present in an amount from 0.01 to 20% by weight.

5. Composition according to one of the preceding claims, **characterized in that** it additionally comprises 0.001 to 5% by weight of at least one natural or synthetic, nonionic, anionic, amphoteric or cationic polymer.

6. Use of at least one O-hydroxyalkylchitin with monomeric units of the general formula as in Claim 1 in compositions for setting hair.

**Revendications**

1. Produit cosmétique ayant une teneur en au moins une O-hydroxyalkylchitine comportant des motifs monomères de formule générale

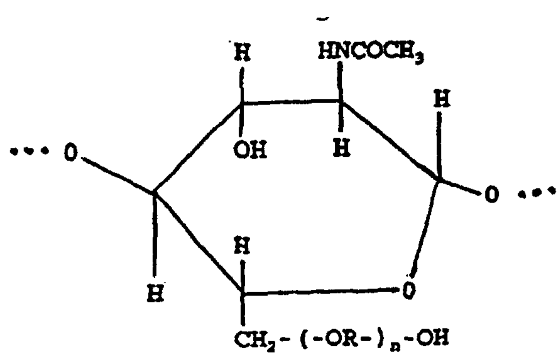

dans laquelle R représente un groupe alkylène linéaire ou ramifié ayant de 3 à 20 atomes de carbone et n représente un nombre égal ou supérieur à 1, dans une base cosmétique appropriée, à l'exclusion d'un produit cosmétique ayant une teneur en une association de (A) N-(3-carboxypropanoyl-6-O-carboxyméthylchitosane et (B) un

dérivé hydrosoluble de chitine choisi parmi l'hydroxypropylchitine et la polyoxyéthylènepolyoxypropylène-chitine, le rapport (A):(B) allant de 9,5:0,5 à 0,5:9,5 et la quantité totale de (A) et (B) allant de 0,001 à 5% en poids.

2. Produit selon la revendication 1, **caractérisé en ce que** R représente un groupe alkylène linéaire ou ramifié ayant de 3 à 6 atomes de carbone.

3. Produit selon l'une quelconque des revendications précédentes, **caractérisé en ce que** n représente un nombre allant de 1,5 à 10.

4. Produit selon l'une quelconque des revendications précédentes, **caractérisé en ce que** l'O-hydroxyalkylchitine est contenue en une quantité allant de 0,01 à 20% en poids.

5. Produit selon l'une quelconque des revendications précédentes, **caractérisé en ce qu'**il contient en outre de 0,001 à 5% en poids d'au moins un polymère non ionique, anionique, amphotère ou cationique, naturel ou synthétique.

6. Utilisation d'au moins une O-hydroxyalkylchitine comportant des motifs monomères de formule générale selon la revendication 1, dans des produits destinés à fixer les cheveux.